Europäisches Patentamt

European Patent Office

Office européen des brevets

(11) Publication number : **0 640 349 A1**

(12) **EUROPEAN PATENT APPLICATION**

(21) Application number : 94306207.5

(22) Date of filing : 23.08.94

(51) Int. Cl.$^6$ : **A61K 39/39, A61K 35/74**

(30) Priority : 25.08.93 JP 230742/93

(43) Date of publication of application :
01.03.95 Bulletin 95/09

(84) Designated Contracting States :
AT BE CH DE DK ES FR GB GR IT LI LU MC NL
PT SE

(71) Applicant : **YAKULT HONSHA CO., LTD.**
1-19, 1-chome Higashishinbashi
Minato-ku
Tokyo (JP)

(72) Inventor : **Yasui, Hisako, c/o K. K. Yakult
Honsha**
1-19 Higashishinbashi 1-chome
Minato-ku, Tokyo (JP)
Inventor : **Kiyoshima, Junko, c/o K. K. Yakult
Honsha**
1-19 Higashishinbashi 1-chome,
Minato-ku
Tokyo (JP)
Inventor : **Ushijima, Hiroshi, 375-5 Gakuencho,
Kodaira-shi,**
Tokyo (JP)

(74) Representative : **Chapman, Paul William et al**
Kilburn & Strode,
30 John Street
London WC1N 2DD (GB)

(54) **Vaccinal efficacy augmentor and efficacy-augmenting foods.**

(57)    A vaccinal efficacy augmentor comprises an IgA production promoter having an IgA production promoting effect as its effective component. The promoter is able to augment the efficacy of pathogenic virus vaccines. When administered with vaccines which contain bacteria or bacilli or virus antigens and which have the purpose of inducing immunity against infectious diseases, the IgA production promoter stimulates IgA producing cells such as Peyer's patch cells to promote secretory IgA production. Eventually, the production of an antibody against the antigen in the vaccine is augmented to well induce protective immunity and augment the efficacy of the vaccine. In addition, the augmentor can suppress side effects of the vaccine. The efficacy augmenting foods comprising the promoter are also described.

EP 0 640 349 A1

## BACKGROUND OF THE INVENTION

### 1. Field of the Invention

This invention relates to an augmentor for vaccinal efficacy comprising, as an effective component, an IgA production promoter, e.g. bacteria belonging to the genus Bifidobacterium, which has a good IgA production augmenting efficacy and also to efficacy-augmenting foods.

### 2. Related Background Art

A number of patients have suffered infectious diseases caused by pathogenic viruses every year not only in developing countries, but also in advanced countries. Especially, such diseases are a main cause for death of infants and young children.

For instance, there have been about several millions of victims including infants every year owing to the rotavirus-infected disease. In Japan, influenza has been vigorously prevailing in every several years. For treatment of these infectious diseases, there have been developed various types of anti-virus agents and vaccines using viral antigens.

However, the development of any appropriate anti-virus agent or vaccine has never been in success for certain types of pathogenic viruses.

The present inventors have already developed a method for searching a substance, which expedites the production of secretory IgA useful for biophylaxy, by use of Pyer's patch cells. Using this method, an IgA production promoter has been found as comprising, as its effective component, bacteria belonging to the genus Bifidobacterium having a good IgA production promoting action (Japanese Laid-open Patent Application No. 280059/1990).

The term "IgA production promoter having the good IgA production promoting action" used herein is intended to mean that in the searching method set out hereinabove, an index ratio (incrementing ratio) of the amount of secretory IgA secreted from IgA producing cells in a culture solution after cultivation over 7 days to the amount of secretory IgA of a non-added group of a substance to be tested (prior to the cultivation) is not smaller than 12.

In order to further augment the IgA production promoting efficacy of the IgA production promoter, an attempt has been made wherein the bifidobacteria are protoplasted to take out the cytoplasmic membrane thereof (Japanese Laid-open Patent Application No. 139469/1991).

## SUMMARY OF THE INVENTION

The present inventors have found that when the bacteria belonging to the genus Bifidobacterium and having a good IgA production promoting action are dosed along with vaccines (pathogenic virus antigen), the production in amount of a specific antibody corresponding to the virus antigen significantly increased, thus augmenting an infection preventive effect.

The object of the present invention is to provide a vaccinal efficacy augmentor wherein when vaccines for pathogenic viruses are administered along with the augmentor, the effect of the vaccine can be augmented, and also to provide efficacy-augmented foods containing the same.

According to a first aspect of the invention, there is provided a vaccinal efficacy augmentor comprising as its effective component an IgA production promoter having an IgA production promoting effect for augmenting the efficacy of a pathogenic virus vaccine, said IgA production promoter comprising a bacterial strain of the genus Bifidobacterium or derivative thereof. Thus, when it is dosed along with vaccines, the production of an antibody corresponding to the antigen present in the vaccine is augmented, resulting in the augmentation of the vaccinal efficacy. It is also possible to suppress side effects of the vaccine.

As is known, vaccines contain bacteria or antigens of viruses and are used to induce immunity against infectious diseases. When an IgA production promoter having the IgA production promoting effect is dosed along with vaccines, the promoter stimulates IgA producing cells such as Pyer's patch cells (PP cells), permitting the production of secretory IgA to be promoted. In the case, the production of an antibody corresponding to the antigen contained in the vaccine is augmented, thereby permitting preventive immunity to be induced and the efficacy of the vaccine to be augmented.

Specific IgA production promoters are those which have a good IgA production promoting effect and which are obtained by the method disclosed in the afore-indicated Japanese Laid-open Patent Application No. 280059/1990, which method is for searching a substance having good capability of inducing the IgA. The method comprises sterilely culturing the PP cells containing a number of IgA producing cells, adding to the culture solution a solution or suspension of a substance to be tested, culturing the resultant mixture over a given period of time, measuring the amount of IgA secreted from the IgA producing cells in the solution, and selecting a substance having the capability of inducing IgA production based on the ratio of the amount of IgA secreted from a group to which the substance has been added, compared to the amount of IgA secreted from a control group.

According to a preferred embodiment of the present invention, the IgA production promoter exhibits an IgA production promoting action such that an index ratio (increasing ratio) of the amount of secretory IgA producing cells in a culture solution after cultivation over 7 days, compared to the amount of secretory IgA secreted from IgA producing cells of a control group, is, for example, not less than 12.

According to another preferred embodiment of the present invention, the specific vaccinal efficacy augmentor comprises, as its effective component, bacteria belonging to the genus Bifidobacterium having a good IgA production promoting efficacy.

Specific examples include human-derived strains such as Bifidobacterium adolescentis, Bifidobacterium angulatum, Bifidobacterium bifidum, Bifidobacterium breve, Bifidobacterium catenulatum, Bifidobacterium gallicum, Bifidobacterium infantis, Bifidobacterium longum, Bifidobacterium pseudocatenulatum, and the like.

Moreover, examples include animal-derived strains such as Bifidobacterium animalis, Bifidobacterium boum, Bifidobacterium choerinum, Bifidobacterium cuniculi, Bifidobacterium dentium, Bifidobacterium gallinarum, Bifidobacterium magnum, Bifidobacterium merycicum, Bifidobacterium minimum, Bifidobacterium pseudolongum subsp. pseudolongum, Bifidobacterium pseudolongum subsp. globosum, Bifidobacterium pullorum, Bifidobacterium ruminantium, Bifidobacterium saeculare, Bifidobacterium suis, Bifidobacterium thermophilum and the like.

Besides, there may be mentioned insect-derived strains such as Bifidobacterium asteroides, Bifidobacterium coryneforme, Bifidobacterium indicum, Bifidobacterium subtile and the like. Needless to say, usable strains are not limited to those set out hereinabove.

Preferred strains belonging to the genus Bifidobacterium include human-derived ones, of which Bifidobacterium longum YIT4062 strain (deposited on April 19, 1989 as FERM No. 2822), Bifidobacterium breve YIT4063 strain (deposited on April 19, 1989 as FERM No. 2823) and Bifidobacterium breve YIT4064 strain (deposited on April 19, 1989 as FERM No. 2824) are more preferred. These strains show a very good augmenting effect on vaccines.

The augmentor for the effect of vaccines according to the invention is considered to show the augmentation as follows:

That is, the IgA production promoter such as a Bifidobacterium strain primarily shows its effect in the intestine in such a way that the effective component of the promoter acts on the PP cells. It is also considered that the resultant IgA is transferred to the whole body, particularly to the systematic mucosal tissues wherein its function is shown. In this sense, the working mechanism of vaccine is not always limited only to the intestine.

However, because the production cells for secretory IgA are present not only in PP cells, but also in mucosal cells, it is considered that a better augmenting action on vaccines is shown for mucous infectious diseases entering from mucous membranes such as oral cavity, respiratory system and alimentary system.

Since the IgA produced with the aid of the promoter of the invention systematically shows its function, it is considered very effective to administer vaccines for infectious diseases, which may include those for percutaneous inoculums, subcutaneous injections, and oral administrations, along with vaccines of the oral administration type, in view of the direct stimulation of PP cells and the ease in administration.

When using percutaneous and subcutaneous vaccines, greater care has to be paid to safety such as on a requirement for a higher degree of purification. With peroral vaccines which have not been put into practice from the standpoint of their efficacy, their combination with the augmentor of the invention may make it possible to provide peroral vaccines which are easier to handle and higher in safety.

Moreover, with regard to infectious diseases for which any vaccine to be clinically applied has not been developed yet, there is considered the high possibility of developing an effective vaccine against the disease when using the augmentor of the invention in combination. In this sense, vaccines which may be used in combination with the augmentor of the invention should not be limited to those currently developed.

The infectious diseases against which the efficacy augmentor of the invention can be applied include ones caused by rotaviruses, polioviruses, influenza viruses, hepatitis A and B viruses, and AIDS viruses which are mucosally infected. Especially, the effects on rotaviruses and influenza viruses are remarkable. Various types of vaccines may be used for the combination, including killed vaccines (inactivated vaccines), vaccines using attenuated strains (viable vaccines), vaccines utilizing part of bacterial structures (component vaccines) and the like.

The strains belonging to the genus Bifidobacterium, which are an effective component of the augmentor of the invention, may be viable or killed. In addition, they may be treated in order to augment their IgA production promoting effect such as by protoplasting, removal of cytoplasmic membrane (Japanese Laid-open Patent Application No. 139469/1991), autolytic treatment (Japanese Laid-open Patent Application No. 46094/1993) and other treatments provided that the effect of the augment is not lost.

The Bifidobacterium strains which are a main component of the augmentor of the invention can be applied to foods such as ordinary fermented milk and are of no safety problem. Accordingly, the amount is not critical and usually ranges from 10 mg to 10 g per dose, with several doses a day. Peroral dosage is most preferred.

According to still another embodiment of the invention, the Bifidobacterium strains are applied in the form of fermented milk or drinks, by which they can be administered prior to or after administration of vaccines to utilize them as a kind of food for augmenting the efficacy of vaccine.

## BRIEF DESCRIPTION OF THE DRAWINGS

Fig. 1 is a graphical representation of the developing rate of diarrhea in relation to the variation in the days after infection for different day old suckling mice;

Fig. 2 is an illustrative view schematically showing a mucous immune mechanism in living body;

Fig. 3 is a graphical representation of the amount of immunoglobulin in serum of mice in relation to the variation in mouse age;

Fig. 4 is an illustrative view showing a test schedule used in an example of the invention;

Fig. 5 is an illustrative view of another test schedule used in another example; and

Fig. 6 is an illustrative view of a further test schedule used in a further example of the invention.

## DESCRIPTION OF THE PREFERRED EMBODIMENTS

Example A (Rotavirus Infection Inhibiting Action)

1. Preparation of an infected mouse model system:

Rotavirus-infected model mice were prepared using rotavirus which could be grown well in MA-104 cells and with which other animal species were very susceptible to be infected and also using BALB/c mice which exhibited the highest IgA induction activity of Bifidobacterium breve (hereinafter abbreviated as B. breve) YIT4064 (FERM Deposit No. 2824).

Rotavirus used was a monkey-derived SA-11 strain (group A, type III). The mice which were infected with rotavirus consisted of suckling mice which were bred and born from 11 to 19 week-old female BALB/c mice and 7 to 15 week-old male BALB/c mice.

Rotavirus was dosed to the suckling mice in such a manner that 4-day-old, 5-day-old, 8-day-old and 12-day-old suckling mice were each orally dosed with rotavirus SA-11 in an amount of $2 \times 10^6$ PFU/20 micro litter, followed by observation of the mice every day as to whether or not they suffered diarrhea.

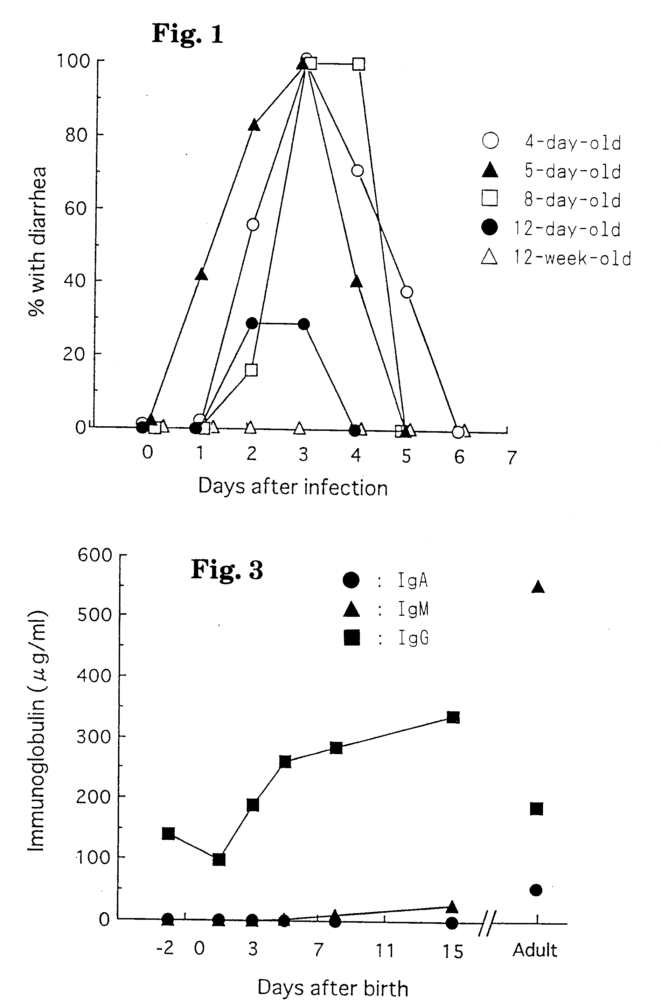

Fig. 1 is a graph showing the developing rate of diarrhea when rotavirus was administered to the respective day-old suckling mice. As shown in the figure, when rotavirus SA-11 was administered to the 4-day-old, 5-day-old and 8-day-old mice each at $2 \times 10^6$ PFU, diarrhea was observed as developed for all the mice at the third day. With the 12-day-old mice, the developing rate of the diarrhea was only at a level of 30 %. The diarrhea of the mice was completely cured at the fifth or sixth day, revealing that the diarrhea was transient.

From the above, it will be seen that the oral administration of SA-11 at $2 \times 10^6$ PFU brings about diarrhea being developed at 100 % for up to 8-day-old suckling mice. Thus, the rotavirus-infected model mice could be provided by infection of 8-day-old or younger suckling mice with rotavirus.

2. Antibody-producing ability of suckling mice:

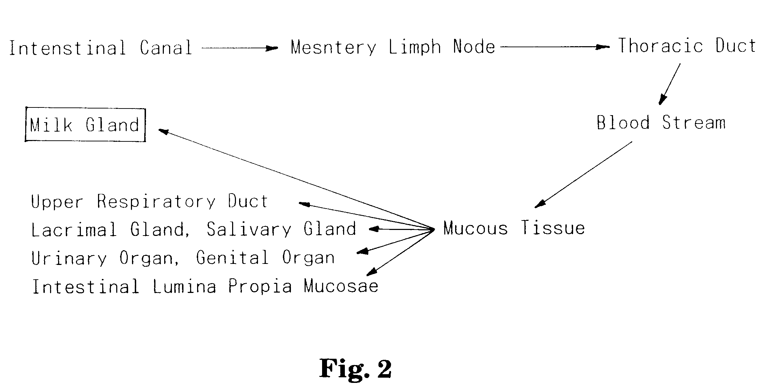

Fig. 2 is an illustrative view which schematically shows the mucous immune mechanism in the body. As is particularly shown in the figure, the respective mucous tissues are correlated with one anther. The cells which are stimulated in the intestine and differentiated into IgA producing cells are transferred toward the mucous tissues such as milk gland, upper respiratory tract and intestinal lamina propria mucosae, thereby secreting secretory IgA.

Accordingly, it is considered that the IgA antibody production augmenting action of B. breve YIT4064 which has the IgA production promoting action is found only in adult mice. The maturation value of the antibody production ability of 8-day-old or younger mice which can be used as infected model mice has been experimentally determined by measurement of immunoglobulin in the serum.

The antibody value was measured according to the ELISA method by which the amounts of anti-SA-11, IgA, IgM and IgG antibodies, and total IgA, total IgM and total IgG antibodies were measured. More particularly, SA-11 which was purified with cesium chloride was coated onto a plate for ELISA using a carbonate buffer (pH 9.6) at a concentration of 0.1 micro grams, to which a sample and a hyperimmune serum were added, followed by addition of the respective classes of enzymatic antibodies. The amounts of the anti-SA-11, IgA, IgM and IgG antibodies were determined from the degree of color development of the substrate. The anti-SA-11 antibody was expressed in terms of the U number wherein a certain value of dilution of the hyperimmune serum was taken as one unit, and the total amount of the antibody was indicated by an absolute value.

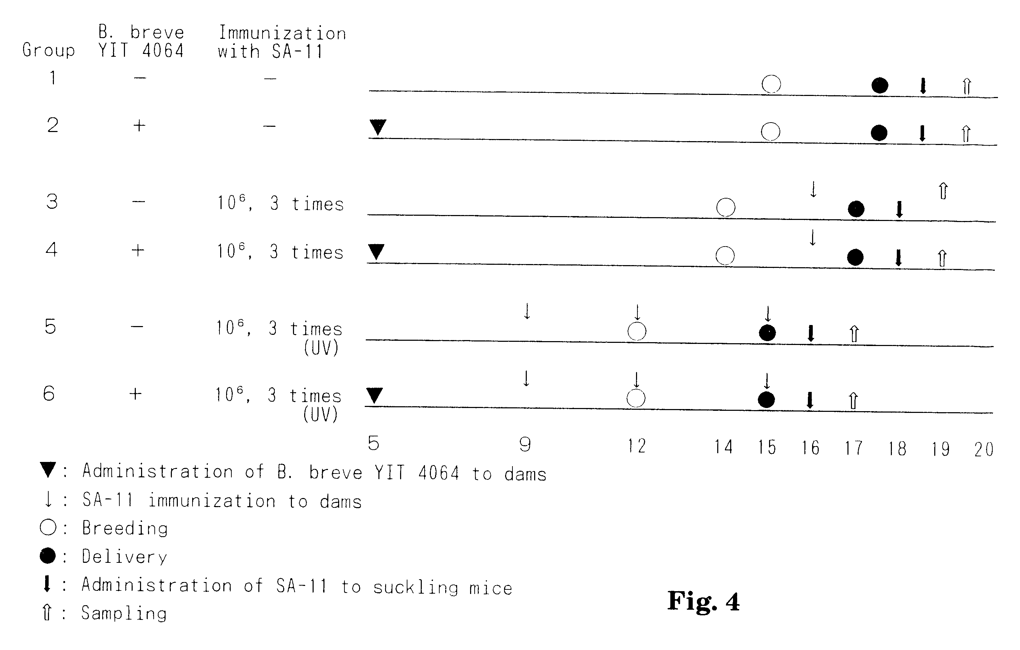

Fig. 3 is a graph showing the amount of immunoglobulin in the serum in relation to the variation in age by days. As shown in Fig. 3, while IgA and IgM were, respectively, detected at a high level in the serum of the dams, these were not detected at all in the suckling mice up to 15-day-old in age. IgG was detected at the time of the birth in an amount equal to that of the dams wherein the amount of IgG of 3-day-old to 15-day-old mice was greater than that of the dams. This is considered for the reason that IgG of the dams is transferred through the placenta and milk.

From the above, it was found impossible to check the infection inhibiting action by direct administration of B. breve YIT4064 to suckling mice. Accordingly, with regard to the effects of administration of vaccines and the Bifidobactrium strain used as a vaccinal effect augmentor, an antigen used as a vaccine was administered to the dams for suckling mice which were used as an infected model. Then, the suckling mice were lactated, which was deemed as having the same effect as of the administration of the vaccine, thereby providing a system for protecting the suckling mice from rotavirus infection.

Example B (Vaccine Augmenting Effect)

1. Passive immunity augmenting effect of B. breve YIT4064:

The protective system against rotavirus infection of the suckling mice was determined by activating the immune mechanism of dams by means of B. breve YIT4064. More particularly, Rotavirus SA-11 (viable cells sterilized by UV irradiation) was inoculated for immunization to dams according to several schedules. The administration of B. breve YIT4064 brought about activation of immunity, and it was checked how the suckling mice lactated from the dams which had been immunized with SA-11 were changed in the development of diarrhea.

Fig. 4 is an illustrative view showing a test schedule in this example. In the figure, there is particularly shown a specific schedule including administration of B. breve YIT4064, immunization, breeding or mating, delivery, administration of SA-11 to suckling mice, and samplings of milk and serum.

Oral administration of B. breve YIT4064 was effected by adding 0.5 % of thermally killed bacteria of B. breve YIT4064 to a feed (MM-3) and permitting the dams to take the feed freely. The dams were immunized by the following three methods, including a method where no immunization was effected on dams (Groups 1 and 2), a method where $1 \times 10^6$ PFU of viable cells off SA-11 was inoculated once (Groups 3, 4) and a method where the cells of $1 \times 10^6$ PFU of SA-11 sterilized by UV irradiation were inoculated three times (Groups 5, 6).

It will be noted that the oral administration of B. breve YIT4064 (0.05 %) was continued over 13 weeks before delivery for Groups 1 to 4. As for Groups 5,6, B. breve YIT4064 was administered over 10 weeks before delivery, followed by continuation of the administration after the delivery. SA-11 ($2 \times 10^6$ PFU) was administered to the 5-day-old suckling mice, followed by observation of the development of diarrhea every day.

Table 1 shows the results of the administration of SA-11 to the suckling mice. As shown in Table 1, a passive protection effect which was produced through the milk of the dams orally administered with B. breve YIT4064 was recognized under all the immunizing conditions. With Group 4, a very high passive protection effect was recognized.

Table 1

Passive protection against SA-11-induced diarrhea in sucking mice bred and fostered from dams orally administered B. breve YIT 4064.

| Group | Immunizing conditions of SA-11 | | B. breve YIT 4064 | Number of Dams | Diarrhea /total | % with diarrhea |
|---|---|---|---|---|---|---|
| 1 | − | *1) | − | 4 | 19/19 | 100.0 |
| 2 | − | *1) | + | 4 | 20/22 | 93.3 |
| 3 | + | *2) | − | 5 | 9/39 | 23.0 |
| 4 | + | *2) | + | 5 | 0/29 | 0 |
| 5 | + | *3) | − | 4 | 24/25 | 96.0 |
| 6 | + | *3) | + | 4 | 16/24 | 66.7 |

*1): No immunization

*2): $1 \times 10^6$ PFU, once, viable bacteria

*3): $1 \times 10^6$ PFU, three times, bacteria killed by UV irradiation

As will be apparent from the above results, the mice suckled with the milk of the dams that took B. breve YIT4064 were more resistant to the rotavirus infection than those mice lactated from the dams that did not take any B. breve YIT4064.

2. Increase of antibody in milk and blood by administration of B. breve YIT4064:

The antibody values and the total amounts of the antibodies in the milk and blood of the dams were measured according to the ELISA Method.

The antibody values and the total amounts of the antibodies of SA-11 in the milk of the dams (nine days after the delivery of the dams which were immunized once through oral administration of $10^6$ PFU of SA-11 prior to 9 to 12 days before the delivery) are shown in Table 2. As shown in Table 2, the IgA antibody value alone of SA-11 significantly increased by the administration of B. breve YIT4064.

Table 2

Antibody titers in milk suckled by suckling mice from darr immunized orally with SA-11 and fed B. breve YIT 4064.

| Antibody class | B. breve YIT 4064 | Anti-SA-11 (U / g) | Total Ig ($\mu$ g / g) |
|---|---|---|---|
| IgA | − (n=16) | 44.6 ± 14.7 | 41.1 ± 32.3 |
| | + (n=11) | 71.7 ± 24.0* | 49.7 ± 26.6 |
| IgM | − | 1.8 ± 1.5 | 23.3 ± 21.2 |
| | + | 1.7 ± 0.6 | 32.9 ± 14.6 |
| IgG | − | 9.9 ± 6.0 | 10.5 ± 5.8 |
| | + | 4.2 ± 1.8 | 12.2 ± 6.1 |

*: $P < 0.05$, n = 5

The antibody values in the blood of the dams (nine days after the delivery of the dams which were immunized once through oral administration of $1\times 10^6$ PFU of SA-11 prior to 9 to 12 days before the delivery) are shown in Table 3. As shown in Table 3, it was not recognized that the antibody values of SA-11 significantly increase through administration of B. breve YIT4064 for the respective classes (IgA, IgM, IgG).

Table 3

| Antibody titers in serum of suckling mice bred and fostered from dams immunized with SA-11 and fed. B. breve YIT 4064. | | | |
|---|---|---|---|
| Antibody class | B. breve YIT 4064 | Anti-SA-11 (U / g) | Total Ig (µg / g) |
| IgA | - | 212.0 ± 57.1 | 299.1 ± 60.2 |
| | + | 268.2 ± 118.1 | 249.3 ± 84.0 |
| IgM | - | 123.9 ± 17.8 | 1543.9 ± 480.1 |
| | + | 117.9 ± 23.4 | 1277.4 ± 240.7 |
| IgG | - | 202.1 ± 70.3 | 134.8 ± 19.3 |
| | + | 138.9 ± 21.9 | 111.1 ± 14.2 |

From the above, it will be seen that the oral administration of B. breve YIT4064 to the dams results in the increase of the IgA antibody value of SA-11 in the milk.

Example C (Effect on Influenza Virus):

The increasing rates of antibodies against influenza virus were measured by an in vitro test system using the murine Peyer's patch (PP) cells.

B. breve YIT4064 (100 µg/mL) was added to the PP cell ($2.5\times 10^6$/mL) cultures along with an influenza virus HA antigen (0, 0.34 or 3.4 micro grams of protein/ml), followed by centrifugal separation on the next day to remove the added influenza virus HA antigen therefrom and cultivation of the PP cells for further 6 days.

The amounts of IgA, IgG and IgM in the resultant supernatant fluid obtained after the cultivation over 6 days were measured by the ELISA method. The results are shown in Table 4.

7

Table 4

Augmentation of anti-influenza virus HA antibody produced by mouse PP cells with B. breve YIT 4064.

| Antibody class | Concn. of influenza virus HA ($\mu$ g of protein/mL) | Antibody titer (U/mL) | |
|---|---|---|---|
| | | No adjuvant | B. breve YIT 4064 |
| IgA | 0 | 0.034 ± 0.044 | 0.181 ± 0.069 |
| | 0.34 | 0.119 ± 0.016 | 0.606 ± 0.009* |
| | 3.4 | 0.050 ± 0.009 | 0.830 ± 0.023* |
| IgG | 0 | 0 ± 0.003 | 0 ± 0.009 |
| | 0.34 | 0 ± 0.011 | 0 ± 0.015 |
| | 3.4 | 0 ± 0.009 | 0 ± 0.009 |
| IgM | 0 | 0 ± 0.075 | 13.934 ± 0.368* |
| | 0.34 | 2.774 ± 0.434 | 18.009 ± 0.406* |
| | 3.4 | 2.660 ± 0.226 | 27.849 ± 0.981* |

*: P < 0.001

As shown in Table 4, it was recognized that when B. breve YIT4064 was added to the PP cell cultures, IgA was significantly produced against the influenza virus HA antigen.

Example D (Augmentation on Influenza Peroral Vaccine, in vivo)

In order to prove the influenza peroral vaccine augmenting action of B. breve YIT4064, an influenza HA antibody (control) or an HA antigen and B. breve YIT4064 (4064 administered group) were orally administered to measure the anti-influenza antibody values in the serum, saliva, and nasal and lung washes thereby comparing the measurements with each other.

The experimental procedure was as follows.

Test animals used were five-week-old BALB/c female mice.

B. breve YIT4064 was orally administered in such a way that the mice were allowed to freely take MM-3 feed to which 0.5 % of thermally killed bacteria of B. breve YIT4064 was added. For control, MM-3 feed was used.

The antigen used was an influenza virus hemagglutinin (HA) vaccine (made by Microorganism Research Lab. of Osaka University, Japan). The virus strains of the vaccines and the content of HA in 1 mL of each vaccine are shown in Table 5 below. It will be noted that the content of proteins was 0.3 mg/mL.

Table 5

| | | |
|---|---|---|
| A/Yamagata/32/89 (H1N1) strain* | : | 300 CCA/ml equivalent |
| A/Beiging/352/89 (H3N2) strain** | : | 250 CCA/ml |
| B/Bangkok/163/90 strain | : | 250 CCA/ml |
| | total : | 800 CCA/ml |

  * : Soviet-type A

** : Hong Kong-type A

The antigen was orally administered using a stomach probe. The pernasal administration of the antigen was effected such that 2.5 mg of isomytal soda (made by Nippon Shinyaku Co., Ltd., Kyoto, Japan) was intraperitoneally administered for anesthesia, followed by dropping into the nostril 10 µL of a mixed solution of 1.5 µg of an antigen and 10 µg of cholera toxin B (CTB) (adjuvant).

The sampling was carried out as follows: 4 µg of carbanyl choline (Wako Junyaku Co., Ltd., Japan) was intraperitoneally administered and the resultant saliva was collected as a sample. Moreover, after completion of the anesthesia, the blood was collected from the heart and the nasal and lung washes were also collected.

The schedule was such that in experiment 1 (influenza oral vaccine augmented action of B. breve YIT4064), 150 µg of the HA antigen was orally administered, from which it was checked whether or not the production of antibodies increased through oral administration of B. breve YIT4064.

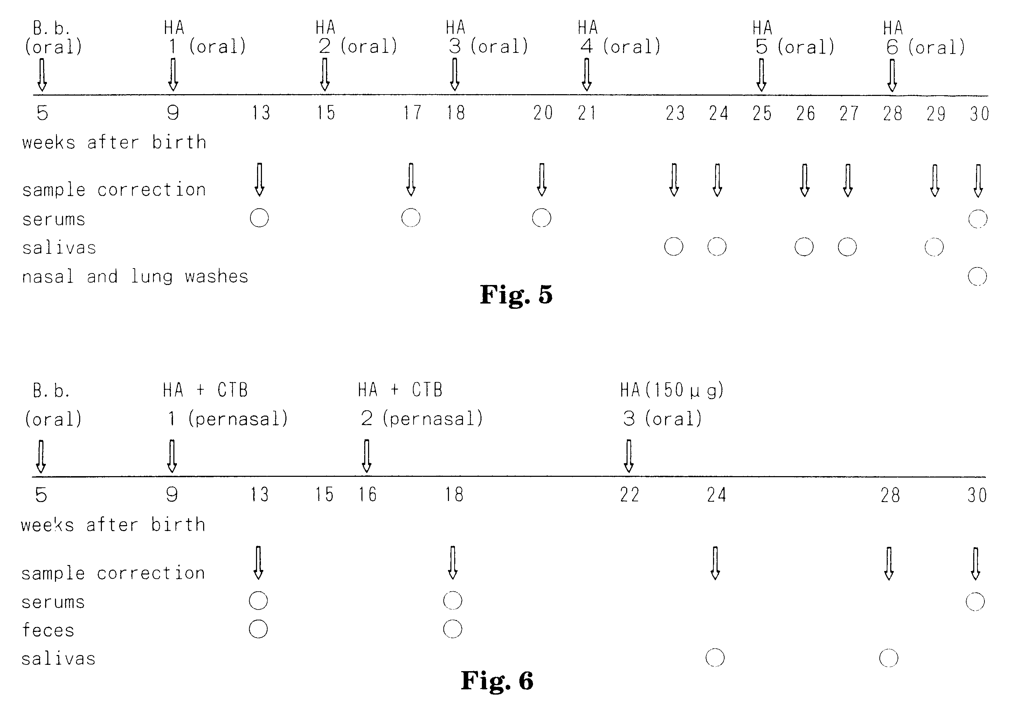

More particularly, as shown in Fig. 5, the oral administration of B. breve YIT4064 started to 5-week-old mice, followed by oral administration of the HA antigen at the ages of 9 weeks, 15 weeks, 18 weeks, 21 weeks, 25 weeks and 28 weeks. On the other hand, the serums of the mice at the ages of 13 weeks, 17 weeks, 20 weeks and 30 weeks were sampled and also salivas were also collected at the ages of 23 weeks, 24 weeks, 26 weeks, 27 weeks and 29 weeks. In addition, at the age of 30 weeks, nasal and lung washes were sampled.

In experiment 2 (influenza pernasal vaccine augmenting action of B. breve YIT4064), 1.5 µg of the HA antigen and 5 µg of CTB are pernasally administered, antibodies are produced. Based on this, it was checked whether or not the oral administration of B. breve YIT4064 could augment pernasal vaccination.

More particularly, as shown in Fig. 6, it was started to orally administer B. breve YIT4064 to 5 week-old mice, followed by pernasal administration of a mixed solution of the HA antigen and CTB at the ages of 9 weeks and 16 weeks and also by oral administration of the HA antigen alone to the 22 week-old mice. On the other hand, the serums were sampled at the ages of 13 weeks, 18 weeks and 30 weeks and the feces were sampled at the ages of 13 weeks and 18 weeks. Additionally, salivas were sampled at the ages of 24 weeks and 28 weeks.

It will be noted that the antibody values in the serums, feces and salivas were measured according to ELISA method.

The IgA anti-HA antibody value is indicated in terms of U unit using a measurement of a 1:200 dilution of the hyper immune serum as 1U. Likewise, the IgG anti-HA antibody is indicated as U unit using a measurement of a 1:200000 dilution of the hyper immune serum as 1U.

The hemagglutination inhibition action was determined by setting such conditions of measuring the hemagglutination inhibition that 25 µL of HA vaccine stepwise diluted 1:2 with a phosphate buffer solution (PBS) containing 0.1 % bovine serum albumin (BSA) and 0.002 % gelatin, 25 µL of the buffer solution and 50 µL of 0.2 % human O-group red blood cells were shook and mixed, followed by reaction at room temperature for 3 hours to determine hemagglutination. The final diluent wherein hemagglutination was recognized was a 1:256000 dilution and was taken as 1 HAu. For the hemagglutination inhibition test, an HA antigen with 4 HAu (1:64000 dilution) was used. The HA vaccine (4 HAu) was added to 25 µl of the 1:2 stepwise diluted sample, followed by reaction at 37 °C for 1 hour, further addition of 50 µL of the 0.2 % human O-group red blood cells and further reaction overnight at room temperature to judge hemagglutination inhibition.

Table 6 shows the results, revealing the influence of B. breve YIT4064 on the production of the anti-influenza HA antibodies. As shown in Table 6, the IgG anti-HA antibody values in the serums of the B. breve YIT4064-administered group significantly increase after the administration three and six times over those values of the control group. However, no difference in the IgA anti-HA antibody values in the serums after the six times of the administration was found between the groups even after administration six times.

Table 6

| Antibody class | Administ-rations | Antibody titer (U/mL) | |
|---|---|---|---|
| | | Control    (n=10) | YIT4064  (n=10) |
| IgA | 1 | 8.9 ±    7. | 10.5 ±      7.6 |
| | 2 | 10.1 ±    9.6 | 12.8 ±      4.4 |
| | 3 | 32.0 ±   21.0 | 48.0 ±     33.0 |
| | 6 | 178.0 ± 195.0 | 260.0 ±    189.0 |
| IgG | 1 | 53.0 ± 157.0 | 32.0 ±     52.0 |
| | 2 | 132.0 ± 309.0 | 305.0 ±    348.0 |
| | 3 | 560.0 ± 754.0 | 1651.0 ± 1342.0* |
| | 6 | 754.0 ± 622.0 | 3876.0 ± 2983.0** |

** : $P < 0.02$,   * : $P < 0.05$

Table 7 shows the influence of B. breve YIT4064 on the production of the anti-influenza antibody in the saliva. As shown in Table 7, the IgA anti-HA antibody value in the saliva tends to be higher in the B. breve YIT4064-administered group than in the control group but is greatly varied with little significant difference being recognized.

Table 7

| Antibody class | Administrations | Antibody titer (U/mL) | |
|---|---|---|---|
| | | Control (n) | YIT4064 (n) |
| IgA | 4-1 | 0.2 ± 0.5 ( 9) | 0.7 ± 0.9 ( 9) |
| | -2 | 0.8 ± 0.4 (10) | 2.5 ± 3.2 (10) |
| | 5-1 | 0.7 ± 0.6 (10) | 2.0 ± 1.9 ( 9) |
| | -2 | 1.1 ± 0.8 (10) | 2.4 ± 2.3 (10) |
| | 6 | 0.6 ± 0.6 (10) | 1.2 ± 1.0 (10) |

Further, Table 8 shows the influence of B. breve YIT4064 on the production of the anti-influenza HA antibody of the respective samples after the administration of the antigen six times. As shown in Table 8, the IgA anti-HA antibody value of the B. breve YIT4064-administered group in the nasal wash after administration of the HA antigen six times significantly increases on comparison with the control group. With the IgA anti-HA antibody value in the lung wash, the B. breve YIT4064-administered group tends to increase, but no significant difference is recognized. With regard to the IgG anti-HA antibody in the serum, a significant increase is found owing to the administration of B. breve YIT4064.

Table 8

| Sample | Antibody class | Antibody titer (U/mL) | |
|---|---|---|---|
| | | Control (n) | YIT4064 (n) |
| serum | | | |
| | IgA | 178 ± 195 (10) | 260 ± 189 (10) |
| | IgG | 754 ± 622 ( 7) | 3876 ± 2983** ( 9) |
| lung wash | IgA | 2.08 ± 1.17 ( 9) | 3.25 ± 1.57 (10) |
| nasal wash | IgA | 0.14 ± 0.13 ( 8) | 1.35 ± 1.37* (10) |

** : P < 0.02, * : P < 0.05

As stated above, B. breve YIT4064 is able to increase the production of the antibodies against the HA antigen simultaneously administered orally, along with the augmentation of IgA in the upper respiratory tract and the IgG anti-HA antibody in the serum. B. breve YIT4064 is able to augment the production of antibody even at the time when oral infection takes place naturally, from which it is assumed that B. breve YIT4064 augments protective ability against subsequent infection.

Eexample E (Vaccinal Effect Augmenting Foods)

1. Culture of B. breve YIT4064 strain and collection of cultured bacteria and method for preparing killed cell product:

An YIT4064 strain was inoculated in a medium mainly composed of lactose (5.5 wt%), yeast extract (1.0 wt%) and skim milk powder (1.0 wt%) was anaerobically cultured at 37 °C for 18 hours while adjusting the pH at 5.5 to 6.0, followed by centrifugal separation, washing and collection of the resultant bacteria. The thus collected bacteria were treated at a pH of 8.0 at a temperature of 40 °C to 55 °C for 1 hour or over and autolyzed, followed by heating at 100 °C for 30 minutes and lyophilization to obtain a killed bacterium powder.

2. Method for baking a loaf of bread:

300 g of wheat flour, 4.5 g of table salt, 3 g of sugar, 3 g of lard, 9 g of bread yeast, 10 g of the killed bacterium product and 180 g of water were blended and set in a mold, followed by baking to make a loaf of bifidus bacteria-incorporated bread.

3. Method for making biscuit:

100 g of wheat flour, 10 g of sugar, 18 g of a shortening, lg of table salt, 1.2 g of baking powder, 5 g of invert sugar, and 5 g of the thermally killed bacteria were well blended, pattern drawn and baked in an oven to obtain a bifidus bacteria-incorporated biscuit.

4. Method for making chocolate:

180 g of a chocolate block, 165 g of cacao butter, 430 g of sugar powder, 220 g of non-defatted milk powder, 5 g of lecithin, small amounts of perfumes, and 15 g of the thermally killed bacteria were blended on a hot bath, followed by cooling for solidification to obtain a bifidus bacteria-incorporated chocolate.

5. Method for making drops:

180 g of sugar, 120 g of starch syrup, 3 g of an organic acid, 10 g of the thermally killed bacteria, and small amounts of perfumes and dyestuffs were blended while boiling down at approximately 150 °C, followed by pouring into molds and cooling for solidification to obtain bifidus bacteria-incorporated drops.

6. Method making controlled milk powder:

Skim milk was added to a raw milk and standardized, followed by addition of sugar, minerals and vitamins in appropriate amounts, sterilization, filtration and subjecting to a spray dryer to obtain a milk powder. 5 % of the thermally killed bacteria was added to the milk powder to obtain a bifidus bacteria-incorporated milk powder.

7. Fermented milk:

A Bifidobacterium breve YIT4064 strain was inoculated in a heated milk medium (15 wt% of skim milk, 0.1 wt% of yeast extract) and cultured at 37 °C until the pH of the medium reached 4.6, followed by cooling and homogenization with a homogenizer. On the other hand, a strain of Streptococcus thermofiles was inoculated in a thermally sterilized milk medium (12 wt% of skim milk) and cultured at 37 °C until the pH of the medium arrived at 4.3, followed by cooling with ice and homogenization with a homogenizer. Both mediums and sucrose syrup were mixed at a ratio of 1:3:1 to obtain a yohgurt drink.

## Claims

1. A vaccinal efficacy augmentor comprising as its effective component an IgA production promoter having an IgA production promoting effect for augmenting the efficacy of a pathogenic virus vaccine, said IgA production promoter comprising a bacterial strain of the genus Bifidobacterium or derivative thereof.

2. A vaccinal efficacy augmentor according to claim 1, wherein the said IgA production promoter has such an IgA production promoting effect that an increment ratio of IgA present in a supernatant of a culture medium of Peyer's patch cells to which the bacterial strain has been added, when compared to one where no bacterial strain has been added, is not less than 12.

3. A vaccinal efficacy augmentor according to claim 1 or claim 2, wherein said bacterial strain is selected from the group consisting of those of Bifidobacterium longum YIT4062 strain (FERM Deposit No 2822), Bifidobacterium breve YIT4063 strain (FERM Deposit No 2823) and Bifidobacterium breve YIT4064 (FERM Deposit No 2824).

4. A vaccinal efficacy augmentor according to any one of claims 1 to 3, wherein the infectious disease for said vaccine is a mucous infectious, virus-infected disease.

5. A vaccinal efficacy augmentor according to any one of claims 1 to 3, wherein the infectious disease for said vaccine is a rotavirus-infected disease.

6. A vaccinal efficacy augmentor according to any one of claims 1 to 3, wherein the infectious disease for said vaccine is an influenza virus infected disease.

7. A vaccinal efficacy-augmenting food comprising a vaccinal efficacy augmentor as defined in any one of claims 1 to 6.

8. The use of a vaccinal efficacy augmentor as defined in any one of claims 1 to 6 in the manufacture of a medicament for use in improving or augmenting the efficacy of a pathogenic virus vaccine.

Fig. 1

Fig. 3

Intenstinal Canal ⟶ Mesntery Limph Node ⟶ Thoracic Duct

Blood Stream

Milk Gland

Upper Respiratory Duct
Lacrimal Gland, Salivary Gland
Urinary Organ, Genital Organ
Intestinal Lumina Propia Mucosae

Mucous Tissue

**Fig. 2**

EP 0 640 349 A1

| Group | B. breve YIT 4064 | Immunization with SA-11 |
|-------|-------------------|-------------------------|
| 1 | − | − |
| 2 | + | − |
| 3 | − | $10^6$, 3 times |
| 4 | + | $10^6$, 3 times |
| 5 | − | $10^6$, 3 times (UV) |
| 6 | + | $10^6$, 3 times (UV) |

▼ : Administration of B. breve YIT 4064 to dams

↓ : SA-11 immunization to dams

○ : Breeding

● : Delivery

↓ : Administration of SA-11 to suckling mice

⇑ : Sampling

**Fig. 4**

EP 0 640 349 A1

B.b.
(oral)

HA
1 (oral)

HA
2 (oral)

HA
3 (oral)

HA
4 (oral)

HA
5 (oral)

HA
6 (oral)

5    9    13    15    17    18    20    21    23    24    25    26    27    28    29    30

weeks after birth

sample correction

serums

salivas

nasal and lung washes

**Fig. 5**

B.b.
(oral)

HA + CTB
1 (pernasal)

HA + CTB
2 (pernasal)

HA (150 μg)
3 (oral)

5    9    13    15    16    18    22    24    28    30

weeks after birth

sample correction

serums

feces

salivas

**Fig. 6**

**European Patent Office**

# EUROPEAN SEARCH REPORT

Application Number

## DOCUMENTS CONSIDERED TO BE RELEVANT

EP 94306207.5

| Category | Citation of document with indication, where appropriate, of relevant passages | Relevant to claim | CLASSIFICATION OF THE APPLICATION (Int. Cl. 6) |
|---|---|---|---|
| X | EP - A - 0 394 136 (KABUSHIKI KAISHA YAKULT HONSHA) * Totality * | 1-8 | A 61 K 39/39 A 61 K 35/74 |
| A | PATENT ABSTRACTS OF JAPAN, unexamined applications, C section, vol. 17, no. 195, April 16, 1993 THE PATENT OFFICE JAPANESE GOVERNMENT page 10 C 1049; & JP-A-04 342 533 (YAKULT HONSHA CO LTD) | 1-8 | |
| P,A | PATENT ABSTRACTS OF JAPAN, unexamined applications, C section, vol. 18, no. 251, May 13, 1994 THE PATENT OFFICE JAPANESE GOVERNMENT page 26 c 1199; & JP-A-06 32 743 (MORINAGA MILK IND CO LTD) | 1-8 | TECHNICAL FIELDS SEARCHED (Int. Cl. 6) A 61 K |

The present search report has been drawn up for all claims

| Place of search | Date of completion of the search | Examiner |
|---|---|---|
| VIENNA | 28-10-1994 | SCHNASS |